# DEMANDE DE BREVET EUROPEEN

(11) **EP 0 628 318 A1**
(43) Date de publication de la demande: **14.12.1994**
(21) Numéro de dépôt: 94401224.4
(22) Date de dépôt: 03.06.1994
(51) Int. Cl.: A61L 11/00, A61L 9/01

(54) **Procédé de désodorisation de déjections animales, déjections animales désodorisées et leur utilisation**

(30) Priorité: 07.06.1993 FR 9306790
(71) Demandeur: S.E.P.P.I.C., SOCIETE D'EXPLOITATION DE PRODUITS POUR LES INDUSTRIES CHIMIQUES, F-75007 Paris (FR)
(72) Inventeur: Le Rouzic, Daniel, F-95120 Ermont (FR)
(74) Mandataire: Caen, Thierry Alain

(57) **Abrégé**

L'invention concerne un procédé de désodorisation de déjections animales selon lequel on met en contact lesdites déjections avec une composition aqueuse comprenant du peroxyde d'hydrogène, au moins un peroxyacide organique et au moins un acide minéral ; déjections animales désodorisées et leur utilisation.

## Description

La présente invention concerne un procédé de désodorisation de déjections animales, des déjections animales désodorisées et leur utilisation en tant qu'engrais agricoles.

Les déjections animales, notamment celles émises par des animaux élevés dans des espaces confinés, se présentent habituellement sous forme de suspensions aqueuses de matières fécales, mélangées ou non avec les urines. Ces suspensions peuvent être plus ou moins diluées avec de l'eau, provenant généralement de l'eau de pluie ou de l'eau de lavage desdits espaces confinés. Des suspensions aqueuses de matières fécales contenant des urines sont généralement désignées sous le terme de lisier.

De telles déjections animales peuvent être collectées et stockées, avant d'être utilisées comme engrais. A cet effet, les déjections animales sont épandues sur de grandes surfaces, généralement à l'aide de dispositifs d'aspersion, tels que des "tonnes à lisier" constituées d'une citerne tractée pourvue de moyens de dispersion au sol des déjections.

L'épandage effectué dans de telles conditions augmente la surface de contact air/liquide entre ces déjections et l'air, ce qui à pour conséquence de provoquer d'importantes nuisances olfactives. Les odeurs émanant de ces déjections animales sont dues à de très nombreux composés chimiques de nature différente, tels des mercaptans, des sulfures, des amines, des acides gras volatils et des cétones. Après épandage, les odeurs émises par les déjections animales peuvent persister pendant plusieurs jours.

En vue de réduire ces odeurs, il a déjà été proposé de traiter ces déjections animales avec des solutions de peroxyde d'hydrogène ou avec des solutions très concentrées d'autres peroxydes tels l'acide péracétique.

Il a été également proposé dans le brevet américain 3.966.450 de contrôler l'odeur émise par des suspensions de matières fécales animales au moyen de peroxyde d'hydrogène, suivi de l'ajustement du pH de ladite suspension par un acide minéral fort.

Cependant de tels traitements ne permettent que de diminuer plus ou moins les odeurs émises, cela, le plus souvent de manière provisoire, et non d'éliminer complètement et définitivement ces odeurs.

En vue d'éliminer plus durablement et plus complètement les odeurs émises par les déjections animales, il a été proposé dans le brevet américain 4.160.656, un procédé de désodorisation qui consiste à traiter les déjections animales à l'état liquide au moyen d'un mélange de formaldéhyde et d'un peroxyde tel le peroxyde d'hydrogène, le percarbonate de sodium ou de potassium, le peroxydisulfate d'ammonium, de sodium ou de potassium, ou des acides percarboxyliques tels les acides peracétique ou perpropionique.

Cependant, la stabilité dans le temps d'un tel mélange de formaldéhyde et de peroxyde est très courte ; elle est en effet de 20 jours au maximum. Plus particulièrement, un mélange d'acide péracétique et de formaldéhyde se décompose au bout de quelques minutes seulement, généralement au bout de 10 à 15 minutes. De plus, cette décompositon est très exothermique, entrainant des risques non négligeables pour la sécurité de l'utilisateur. En raison de cette très grande instabilité, le brevet américain 4.160.656 préconise de ne préparer le mélange de formaldéhyde et de peroxyde que quelques minutes avant son utilisation pour désodoriser les déjections animales.

De plus, le formaldéhyde étant un composé relativement toxique, on comprend que les déjections traitées avec un tel mélange ne peuvent être épandues sans risque pour l'environnement.

Un premier objet de l'invention consiste alors en un procédé pour désodoriser les déjections animales de façon complète et définitive.

Un autre objet de l'invention consiste en un procédé de désodorisation de déjections animales de mise en oeuvre simple et sans danger pour la sécurité de l'utilisateur, pouvant être effectué au moyen d'une composition stable dans le temps et qui ne requiert pas d'être préparées dans les jours, voire les heures ou les minutes précédant son utilisation.

Un troisième objet de la présente invention consiste en des déjections animales désodorisées.

Encore un autre objet de la présente invention consiste en l'utilisation de déjections animales désodorisées comme engrais agricoles, sans risque pour l'environnement.

L'invention consiste en un procédé de désodorisation de déjections animales caractérisé en ce que l'on met en contact lesdites déjections animales avec une composition aqueuse comprenant du peroxyde d'hydrogène, au moins un peroxyacide organique et au moins un acide minéral.

Le peroxyacide organique peut être avantageusement un composé de formule R - CO₃H, R étant un radical alkyle, linéaire ou ramifié, comportant 1 à 25 atomes de carbone et de préférence 1 à 11 atomes de carbone.

A titre de composé de formule R-CO₃H, on peut citer l'acide perpropionique. De manière particulièrement avantageuse dans le cadre de la présente invention, le peroxyacide est l'acide peracétique.

L'acide minéral peut être l'acide sulfurique ou de préférence l'acide nitrique, l'acide phosphorique, l'acide chlorhydrique ou un mélange d'au moins deux de ces acides. L'acide nitrique est un acide minéral particulièrement préféré.

Ladite composition aqueuse comporte habituellement, en poids, de 5 à 3 5 % de peroxyde d'hydrogène, de 1 à 20 % de peroxyacide organique et de 2 à 15 % d'acide minéral. Plus particulièrement, cette composition aqueuse comporte, en poids, de 10 à 25 % de peroxyde d'hydrogène, de 1 à 5 % de peroxyacide et de 3 à 10 % d'acide minéral.

En outre, cette composition aqueuse peut comporter un acide organique. Celui-ci est de préférence l'acide organique à partir duquel on prépare de manière classique, ledit peroxyacide organique. Lorsque le peroxyacide organique est un composé de formule R-CO₃H, l'acide organique est donc de préférence un acide carboxylique de formule R'-COOH, R' étant identique à R. Ainsi, si le peroxyacide est l'acide péracétique, l'acide carboxylique compris dans ladite composition aqueuse est l'acide acétique. La teneur en poids de cet acide organique peut être compris entre 2 et 10 %, de préférence entre 3 et 8 % par rapport au poids total de la composition aqueuse.

Par ailleurs, la composition aqueuse peut comporter des stabilisants usuels du peroxyde d'hydrogène tels des composés phosphoniques, des stanates ou des pyrophosphates.

Une composition aqueuse préférée consiste en celle décrite dans la demande de brevet européen 87.343.

Les procédés de préparations de ces compositions aqueuses sont bien connus de l'homme du métier. Ainsi, ces compositions peuvent être préparées par simple mélange des composés qui les constituent. Par ailleurs, de telles compositions aqueuses sont disponibles dans le commerce, comme celle commercialisée par la Société Seppic, France, sous la désignation BACTIPAL (marque déposée).

La mise au contact entre les déjections animales et ladite composition aqueuse peut se faire par tout moyen conventionnel. Cette mise au contact peut notamment se faire par simple mélange entre la composition aqueuse et les déjections animales. Habituellement, les déjections animales sont stockées dans des cuves spécialement aménagées à cet effet. En vue de leur épandage, les déjections animales peuvent être acheminées de leur cuve de stockage vers une citerne tractée telle une tonne à lisier. Cet acheminenent est généralement effectué au moyen d'un tuyau d'alimentation reliant la cuve de stockage et la citerne tractée, celle-ci étant mise en dépression. En vue de la mise en contact des déjections animales et de la composition aqueuse, on peut, dans un premier temps, introduire une quantité déterminée de la composition aqueuse dans la citerne, puis dans un second temps, y introduire les déjections animales. Un mélange efficace et homogène peut être ainsi réalisé entre les déjections animales et ladite composition aqueuse, ce qui conduit à une désodorisation complète et définitive de ces déjections animales.

Généralement, on met en contact de 0,1 à 2 litres, de préférence de 0,4 à 1 litre, de composition aqueuse par m³ de déjections animales.

Les déjections animales pouvant être traitées selon l'invention, consistent le plus souvent en une suspension aqueuse de matières fécales pouvant contenir le cas échéant des urines. Plus particulièrement, les déjections animales peuvent consister en un lisier, notamment un lisier de porc. Ces types de suspensions peuvent, éventuellement, être diluées par de l'eau.

Par ailleurs, les matières solides en suspension peuvent être liquifiées de manière classique, par exemple au moyen de micro-organismes ; celà préalablement à la mise en oeuvre du procédé de désodorisation selon l'invention.

L'invention concerne également des déjections animales désodorisées qui sont susceptibles d'être obtenues selon le procédé décrit ci-dessus.

Ces déjections animales désodorisées peuvent être utilisées efficacement comme engrais agricole. En effet, la composition aqueuse mise en oeuvre pour désodoriser ces déjections animales contient des acides minéraux tels l'acide nitrique ou l'acide phosphorique, qui, au contact des déjections animales, se transforment en sels, tels des nitrates et des phosphates.

L'exemple qui suit a pour but d'illustrer la présente invention.

### Exemple 1

On introduit dans une tonne à lisier une composition aqueuse ① comprenant (% en poids) :
- peroxyde d'hydrogène 18 %
- acide peracétique 2,5 %
- acide acétique 5,5 %
- acide nitrique 8 %
- stabilisant 0,4 %
- eau qsp 100

① composition aqueuse commercialisée par la Société Seppic, France, sous la marque déposée BACTIPAL.

On introduit ensuite dans la tonne a lisier, du lisier de porc.

Le volume en la composition aqueuse décrite ci-dessus introduit dans la tonne à lisier, est prédéterminé de sorte que 0,5 litre de cette composition aqueuse soit mise en contact avec un m³ de lisier de porc.

On réalise ainsi une mise au contact homogène entre ladite composition aqueuse et le lisier de porc.

Dans les 2 heures qui suivent le remplissage de la tonne à lisier, on procède à l'épandage de ce lisier de porc mis au contact avec la composition aqueuse. Lors de cet épandage aucune odeur n'a pu être détectée. De même, quatre jours après cet épandage, aucune odeur nauséabonde n'a été émise.

## Revendications

1. Procédé de désodorisation de déjections animales caractérisé en ce qu'on met en contact lesdites déjections avec une composition aqueuse comprenant du peroxyde d'hydrogène, au moins un peroxyacide organique et au moins un acide minéral.

2. Procédé selon la revendication 1 caractérisé en ce que le peroxyacide organique est un composé de formule R-CO₃H, R étant un radical alkyle, linéaire ou ramifié, comportant 1 à 25 atomes de carbone et de préférence 1 à 11 atomes de carbone.

3. Procédé selon la revendication 2 caractérisé en ce que le peroxyacide est l'acide péracétique.

4. Procédé selon l'une des revendications précédentes caractérisé en ce que l'acide minéral est l'acide nitrique, l'acide sulfurique, l'acide phosphorique, l'acide chlorhydrique ou un mélange d'au moins deux de ces acides.

5. Procédé selon la revendication 4 caractérisé en ce que l'acide minéral est l'acide nitrique.

6. Procédé selon l'une des revendications précédentes caractérisé en ce que ladite composition aqueuse comporte, en poids, de 5 à 35 % de peroxyde d'hydrogène, de 1 à 20 % de peroxyacide et de 2 à 15 % d'acide minéral.

7. Procédé selon la revendication 6 caractérisé en ce que ladite composition aqueuse comporte, en poids, de 10 à 25 % de peroxyde d'hydrogène, de 1 à 5 % de peroxyacide et de 3 à 10 % d'acide minéral.

8. Procédé selon l'une des revendications précédentes caractérisé en ce que la composition aqueuse comporte en outre un acide organique, tel un acide carboxylique de formule R-COOH, R' étant identique à R.

9. Procédé selon l'une des revendications précédentes caractérisé en ce qu'on met au contact de 0,1 à 2 1, de préférence de 0,4 à 1 1 de ladite composition aqueuse par m³ de déjections animales.

10. Procédé selon l'une des revendications précédentes caractérisé en ce que les déjections animales consistent en une suspension aqueuse de matières fécales et le cas échéant, d'urines.

11. Procédé selon la revendications 10 caractérisé en ce que les déjections animales consistent en un lisier de porc.

12. Déjections animales désodorisées caractérisées en ce qu'elles sont susceptibles d'être obtenues selon le procédé de l'une des revendications 1 à 11.

13. Utilisation de déjections animales désodorisées selon la revendication 12 comme engrais agricole.
